# EUROPEAN PATENT SPECIFICATION

(11) **EP 2 992 111 B1**
(45) Date of publication and mention of the grant of the patent: **26.12.2018**
(21) Application number: 14723469.4
(22) Date of filing: 30.04.2014
(51) Int. Cl.: C12Q 1/6883, G01N 33/68

(54) **DIFFERENTIALLY EXPRESSED BIOMARKERS FOR ALZHEIMER'S DISEASE**
UNTERSCHIEDLICH EXPRIMIERTE MARKER FÜR DIE ALZHEIMER KRANKHEIT
MARQUEURS DIFFÉRENTIELLES EXPRIMÉS POUR LA MALADIE D`ALZHEIMER

(30) Priority: 03.05.2013 GB 201308077
(43) Date of publication of application: 09.03.2016
(73) Proprietor: CompanDX Limited, Nottingham NG1 1GF (GB)
(72) Inventor: BALL, Graham, Nottingham NG5 8HL (GB)
(74) Representative: Potter Clarkson LLP
(86) International application number: PCT/GB2014/051346
(87) International publication number: WO 2014/177867

(56) References cited:
- JP-A- 2005 132 738
- US-A1- 2005 289 657
- US-A1- 2008 269 103
- US-A1- 2009 275 495
- KONG WEI ET AL: "Independent component analysis of Alzheimer's DNA microarray gene expression data", MOLECULAR NEURODEGENERATION, BIOMED CENTRAL LTD, LO, vol. 4, no. 1, 28 January 2009 (2009-01-28), page 5, XP021052336, ISSN: 1750-1326, DOI: 10.1186/1750-1326-4-5
- SULTANA RUKHSANA ET AL: "Proteomics analysis of the Alzheimer's disease hippocampal proteome", JOURNAL OF ALZHEIMER'S DISEASE, vol. 11, no. 2, 2007, pages 153-164, XP009179136, ISSN: 1387-2877 [retrieved on 2007-05-21]
- GUERREIRO NELSON ET AL: "Proteomic 2-D DIGE profiling of APP23 transgenic mice brain from pre-plaque and plaque phenotypes", JOURNAL OF ALZHEIMER'S DISEASE, IOS PRESS, NL, vol. 13, no. 1, 1 February 2008 (2008-02-01), pages 17-30, XP009179164, ISSN: 1387-2877

## Description

The present invention relates to novel biomarkers for determining the Alzheimer's disease status of a subject, and to uses of novel panels of biomarkers.

The rising global incidence of Alzheimer's disease (AD) poses an increasingly profound problem to society yet progress towards a genuine understanding of the disease remains worryingly slow. As individual life expectancy increases, the incidence of AD worldwide predicted to rise from an estimated 35.6 million people in 2010 to 65.7 million people by 2030.

Perhaps the most outstanding problem with the biology of AD is the question of its mechanistic origins as it remains unclear where the molecular failures occur that underlie the causes and progression of the disease. In other words, changes in the activity or behaviour of (e.g.) specific biochemical pathways which ultimately lead to the cellular damage and neurological disfunction remain unclear. Accordingly, definitions of AD based on the characteristic hallmarks of disease such as amyloid plaques and neurofibrillary tangles associated with brain lesions and cell death represent consequences of the disease rather than the cause.

Currently AD diagnosis is only confirmed post-mortem by the presence of pathological hallmarks including plaques mainly composed of aggregated amyloid-beta (Aβ) and neurofibrillary tangles (NFT) of hyperphosphorylated tau protein. AD is currently clinically diagnosed using tests of cognitive function. Studies have shown that the accuracy of the clinical diagnosis is between 65 and 90% using cognitive techniques. Higher accuracy is achieved at academic centres with special interest in AD. However, this is only at a late stage of the disease when there is extensive irreversible neuronal damage already present.

AD pathology often accumulates over a 10 to 20 year period before clinical symptoms present and even patients with mild cognitive impairment (MCI) have been found to display marked AD pathology at PM.

KONG WEI ET AL: "Independent component analysis of Alzheimer's DNA microarray gene expression data", MOLECULAR NEURODEGENERATION, BIOMED CENTRAL LTD, LO, vol. 4, no. 1, 28 January 2009, page 5,

SULTANA RUKHSANA ET AL: "Proteomics analysis of the Alzheimer's disease hippocampal proteome", JOURNAL OF ALZHEIMER'S DISEASE, vol. 11, no. 2, 2007, pages 153-164,

GUERREIRO NELSON ET AL: "Proteomic 2-D DIGE profiling of APP23 transgenic mice brain from pre-plaque and plaque phenotypes", JOURNAL OF ALZHEIMER'S DISEASE, IOS PRESS, NL, vol. 13, no. 1, 1 February 2008 (2008-02-01), pages 17-30,

JP 2005 132738 A (PROTEIN EXPRESS KK; SHIMOHAMA TAKASHI; TSUJI TERUYUKI) 26 May 2005,

US 2009/275495 A1 (WARD MALCOLM [GB] ET AL) 5 November 2009 (2009-11-05),
disclose genes or proteins differentially disclosed in Alzheimer's disease.

A reliable, accurate biomarker or set of biomarkers would be an invaluable aid to clinical diagnosis and potentially identify affected individuals much earlier when drug intervention is likely to be most effective. There is an urgent need for reliable and accurate biomarkers to aid clinical diagnosis and progression of Alzheimer's disease (AD).

It would be advantageous to be able to test for the Alzheimer's disease status of an individual subject to distinguish between affected and unaffected individuals, in particular it would be advantageous to be able to test the AD status of an individual at an early stage before symptoms are present or in patients with mild cognitive impairment. Such a test could also be used to follow the AD status of individuals during treatment to determine how the disease is progressing and assess the effect of the treatment.

In a first aspect the present invention provides a method of determining the Alzheimer's disease status of a subject comprising:
(a) providing a sample of material obtained from the subject; and
(b) determining the expression level of TUBB, NEAT1, CENPB, AA482221 and CPSF3 genes in the sample.

**Table 1 Control versus AD probes of high significance**

| | | |
|---|---|---|
| TUBB | Tubulin Beta Class 1 | Cell structure and Transport of proteins into and outside of cell |
| NEAT1 | nuclear paraspeckle assembly transcript 1 | Hypothetical role in the localisation / compartmentalisation of proteins |
| CENPB | Centromere formation | Behaves as a microtubule associated protein and linked to TUBB |
| AA482221 | | Unknown function |
| CPSF3 | Cleavage and polyadenylation specific factor 3 | Plays a key role in pre-mRNA 3'-end formation brings about cleavage and poly(A) addition |

The method may further comprise:
(c) determining the expression level of one or more genes selected from the group consisting of:
seryl-tRNA synthetase
stathmin-like 2
RNA binding motif protein, X-linked
anaphase promoting complex subunit 13
reticulon 3
proteasome (prosome, macropain) subunit, beta type, 3 hypothetical LOC100509179
optineurin
tyrosine 3-monooxygenase/tryptophan 5-monooxygenase activation protein, zeta polypeptide
OCIA domain containing 1
ATPase, H+ transporting, lysosomal 31kDa, V1 subunit E1 fibroblast growth factor (acidic) intracellular binding protein aftiphilin
Berardinelli-Seip congenital lipodystrophy 2 (seipin) glutamic-oxaloacetic transaminase 1, soluble (aspartate aminotransferase 1)
ATPase, Ca+ + transporting, cardiac muscle, slow twitch 2 DAZ interacting protein 3, zinc finger
CAP, adenylate cyclase-associated protein 1
tubulin, alpha 1b
NADH dehydrogenase (ubiquinone) 1 alpha subcomplex, 7, 14.5kDa
eukaryotic translation initiation factor 2B, subunit 3 gamma, 58kDa
ribosomal protein L15
tubulin, alpha 1b
proteasome (prosome, macropain) 26S subunit, non-ATPase, 1 proteasome (prosome, macropain) subunit, beta type, 2
ATP synthase, H+ transporting, mitochondrial F1 complex, gamma polypeptide 1
tetratricopeptide repeat domain 15
processing of precursor 4, ribonuclease P/MRP subunit (S. cerevisiae)
rabaptin, RAB GTPase binding effector protein 1.

**Table 2 Control versus AD probes of significance**

| | |
|---|---|
| 1 | seryl-tRNA synthetase |
| 2 | stathmin-like 2 |
| 3 | RNA binding motif protein, X-linked |
| 4 | anaphase promoting complex subunit 13 |
| 5 | reticulon 3 |
| 6 | proteasome (prosome, macropain) subunit, beta type, 3 |
| 7 | hypothetical LOC100509179 |
| 8 | optineurin |
| 9 | tyrosine 3-monooxygenase/tryptophan 5-monooxygenase activation protein, zeta polypeptide |
| 10 | OCIA domain containing 1 |
| 11 | ATPase, H+ transporting, lysosomal 31kDa, V1 subunit E1 |
| 12 | fibroblast growth factor (acidic) intracellular binding protein |
| 13 | aftiphilin |
| 14 | Berardinelli-Seip congenital lipodystrophy 2 (seipin) |
| 15 | glutamic-oxaloacetic transaminase 1, soluble (aspartate aminotransferase 1) |
| 16 | ATPase, Ca+ + transporting, cardiac muscle, slow twitch 2 |
| 17 | DAZ interacting protein 3, zinc finger |
| 18 | CAP, adenylate cyclase-associated protein 1 |
| 19 | tubulin, alpha 1b |
| 20 | NADH dehydrogenase (ubiquinone) 1 alpha subcomplex, 7, 14.5kDa |
| 21 | eukaryotic translation initiation factor 2B, subunit 3 gamma, 58kDa |
| 22 | ribosomal protein L15 |
| 23 | tubulin, alpha 1b |
| 24 | proteasome (prosome, macropain) 26S subunit, non-ATPase, 1 |
| 25 | proteasome (prosome, macropain) subunit, beta type, 2 |
| 26 | ATP synthase, H+ transporting, mitochondrial F1 complex, gamma polypeptide 1 |
| 27 | tetratricopeptide repeat domain 15 |
| 28 | processing of precursor 4, ribonuclease P/MRP subunit (S. cerevisiae) |
| 29 | rabaptin, RAB GTPase binding effector protein 1 |

Determination of the level of a biomarker in a sample may comprise the detection of a polypeptide or an mRNA with at least 65% sequence identity, more preferably at least 70%, 75%, 80%, 85%, 90%, 95% or greater sequence identity, to the published sequence of the biomarker, for example the published sequence of a gene listed in Table 1 or Table 2 or the published sequence of a protein encoded by a gene listed in Table 1 or Table 2.

The method may further comprise:
(d) employing the expression level determined in (b) and optionally (c) to distinguish between healthy subjects and subjects having Alzheimer's disease.

According to the present disclosure, the expression level of one, two, three, four or all five genes from Table 1 may be tested in a sample in order to provide an indication that is useful for diagnosing Alzheimer's disease. The more genes that are tested the higher the accuracy of the test.

Where 1 gene selected from Table 1 is used the test may be 84% accurate for predicting Alzheimer's disease in a subject. Where 2 genes selected from Table 1 is used the test may be 86.6 % accurate for predicting Alzheimer's disease in a subject. Where 3 genes selected from Table 1 are used the test may be 88.6% accurate for predicting Alzheimer's disease in a subject. Where 4 genes selected from Table 1 are used the test may be 92.8% accurate for predicting Alzheimer's disease in a subject. Where 5 genes selected from Table 1 are used the test may be 94.85% accurate for predicting Alzheimer's disease in a subject.

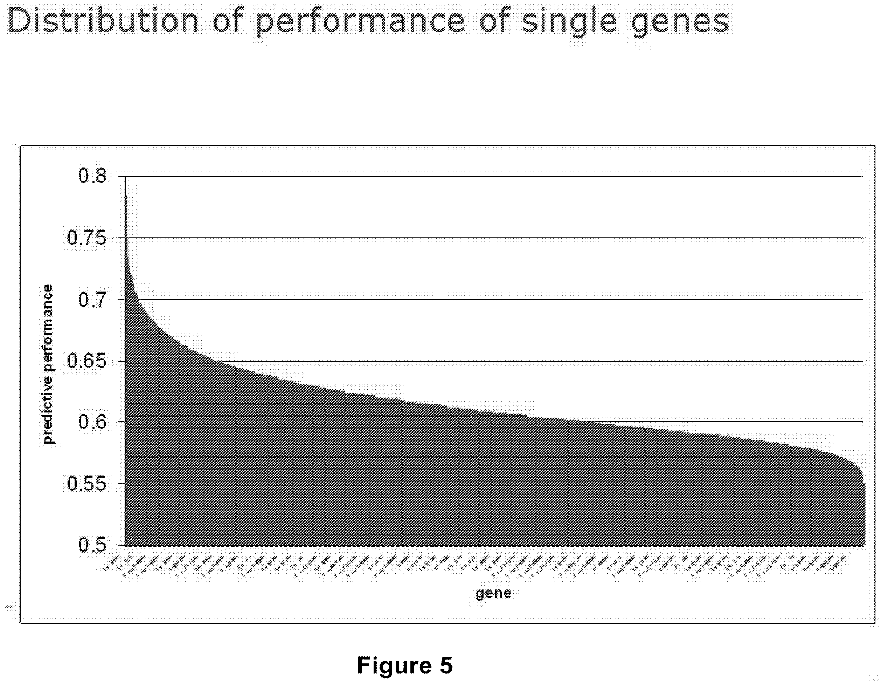

The accuracies of each gene from Table 2 are shown in Figure 5.

Expression level of any one of the genes from Table 1 and any one or more of the genes in Table 2 may be tested to provide an indication that is useful for diagnosing Alzheimer's disease.

Expression level of two, three, four, or five genes from Table 1 and at least one gene from Table 2 may be tested to provide an indication that is useful in diagnosing Alzheimer's disease.

Expression level of two, three, four, or five genes from Table 1 and at least one gene from Table 2 may be tested to provide an indication that is useful in diagnosing Alzheimer's disease.

The expression level of one or more genes selected from the group in Table 1 and optionally one or more genes selected from Table 2 in a sample may be determined by any suitable method. Such methods include methods that quantify the nucleotide products of these genes such as: quantitative PCR using suitable oligonucleotide primers designed to adhere within the sequence of an mRNA encoded by the gene of interest; analysis of expression arrays; next generation sequencing, comparative genomic hybridisation arrays (CGH arrays); multiplexed PCR. The expression level of one or more genes selected from Table 1 an optionally one or more of the genes selected from Table 2 in a sample may also be determined using methods that quantify the protein products of the selected genes, for example: ELISA; immunohistochemistry; protein aptamer arrays or protein immunological arrays.

The expression level of the gene may be determined by measuring the rate of polymerisation of the RNA using standard techniques.

The method disclosed may not include the step of obtaining the sample.

The subject may be a mammal, and is preferably a human, but may alternatively be a monkey, ape, cat, dog, cow, horse, rabbit or rodent.

The expression level determined in (b) and optionally (c) may be compared to a standard.

The standard may the expression level that would be expected for the same gene in a healthy individual. For example the expression level of a gene in a sample may be compared to the expression level of the same gene in a comparable sample of the same tissue in a healthy individual. The expression level in a sample may be compared to the expected level in a comparable sample from a healthy individual.

The method of the invention may include a further step of comparing the determined value of the expression level of one or more genes selected from Table 1 or Table 2 with a reference value.

The reference value may be the value for the expression level of the same gene in a sample of the same sample type, from an individual who is known to have or not to have Alzheimer's disease. Alternatively, or additionally, the reference value may be the expression level of the same gene in the same sample type in a sample taken previously from the same subject, for example, prior to or during the course of a particular treatment. The reference sample may be a sample of the same type, for example, both samples may be blood samples. In this way the expression level of one or more genes selected from Table 1 or Table 2 may be used to monitor the progression of disease in a subject, and/or to monitor the efficacy of a particular treatment in a subject.

The method disclosed may be carried out *in vitro.*

About a 10% or more increase in expression of a biomarker compared to the level in a normal sample may be diagnostic of Alzheimer's disease.

About a 20% or more increase in expression of a biomarker compared to the level in a normal sample may be diagnostic of Alzheimer's disease.

About a 30% or more increase in expression of a biomarker compared to the level in a normal sample may be diagnostic of Alzheimer's disease.

About a 40% or more increase in expression of a biomarker compared to the level in a normal sample may be diagnostic of Alzheimer's disease.

About a 50% or more increase in expression of a biomarker compared to the level in a normal sample may be diagnostic of Alzheimer's disease.

About a 60% or more increase in expression of a biomarker compared to the level in a normal sample may be diagnostic of Alzheimer's disease.

About a 70% or more increase in expression of a biomarker compared to the level in a normal sample may be diagnostic of Alzheimer's disease.

About a 80% or more increase in expression of a biomarker compared to the level in a normal sample may be diagnostic of Alzheimer's disease.

About a 90% or more increase in expression of a biomarker compared to the level in a normal sample may be diagnostic of Alzheimer's disease.

About a 100% or more increase in expression of a biomarker compared to the level in a normal sample may be diagnostic of Alzheimer's disease.

About a 10% or more decrease in expression of a biomarker compared to the level in a normal sample may be diagnostic of Alzheimer's disease.

About a 20% or more decrease in expression of a biomarker compared to the level in a normal sample may be diagnostic of Alzheimer's disease.

About a 30% or more decrease in expression of a biomarker compared to the level in a normal sample may be diagnostic of Alzheimer's disease.

About a 40% or more decrease in expression of a biomarker compared to the level in a normal sample may be diagnostic of Alzheimer's disease.

About a 50% or more decrease in expression of a biomarker compared to the level in a normal sample may be diagnostic of Alzheimer's disease.

About a 60% or more decrease in expression of a biomarker compared to the level in a normal sample may be diagnostic of Alzheimer's disease.

About a 70% or more decrease in expression of a biomarker compared to the level in a normal sample may be diagnostic of Alzheimer's disease.

About a 80% or more decrease in expression of a biomarker compared to the level in a normal sample may be diagnostic of Alzheimer's disease.

About a 90% or more decrease in expression of a biomarker compared to the level in a normal sample may be diagnostic of Alzheimer's disease.

About a 100% or more decrease in expression of a biomarker compared to the level in a normal sample may be diagnostic of Alzheimer's disease.

In a panel of biomarkers an increase in expression of some biomarkers compared to the level in a normal sample and a decrease in other biomarkers compared to the level in a normal sample may be diagnostic of Alzheimer's disease.

A panel of biomarkers may comprise or consist of TUBB, NEAT1, CENPB, AA482221 and CPSF3. A Decrease in expression of TUBB and an increase in the expression of NEAT1 and an increase in the expression of CENPB and a decrease in the expression of AA482221 and a decrease in expression of CPSF3 may be diagnostic of Alzheimer's disease. This panel of biomarkers may have about 94% or about 94.85% accuracy in predicting Alzheimer's disease.

A panel of biomarkers may comprise or consist of TUBB, NEAT1, CENPB and AA482221. A Decrease in expression of TUBB and an increase in the expression of NEAT1 and an increase in the expression of CENPB and a decrease in the expression of AA482221 may be diagnostic of Alzheimer's disease. This panel of biomarkers may have about 92% or about 92.78% accuracy in predicting Alzheimer's disease.

A panel of biomarkers may comprise or consist of TUBB, NEAT1 and CENPB. A Decrease in expression of TUBB and an increase in the expression of NEAT1 and an increase in the expression of CENPB may be diagnostic of Alzheimer's disease. This panel of biomarkers may have about 88% or about 88.66% accuracy in predicting Alzheimer's disease.

A panel of biomarkers may comprise or consist of TUBB and NEAT1. A Decrease in expression of TUBB and an increase in the expression of NEAT1 may be diagnostic of Alzheimer's disease. This panel of biomarkers may have about 86% or about 86.60% accuracy in predicting Alzheimer's disease.

A panel of biomarkers may comprise or consist of TUBB. A Decrease in expression of TUBB may be diagnostic of Alzheimer's disease. This panel of biomarkers may have about 84% or 84.02% accuracy in predicting Alzheimer's disease.

A panel of biomarkers may comprise or consist of TUBB, NEAT1, CENPB, AA482221 and CPSF3 and one additional biomarker selected from Table 2.

A panel of biomarkers may comprise or consist of TUBB, NEAT1, CENPB, AA482221 and CPSF3 and two additional biomarkers selected from Table 2.

A panel of biomarkers may comprise or consist of TUBB, NEAT1, CENPB, AA482221 and CPSF3 and three additional biomarkers selected from Table 2.

A panel of biomarkers may comprise or consist of TUBB, NEAT1, CENPB, AA482221 and CPSF3 and four additional biomarkers selected from Table 2.

A panel of biomarkers may comprise or consist of TUBB, NEAT1, CENPB, AA482221 and CPSF3 and five additional biomarkers selected from Table 2.

A biomarker panel may be 6 biomarkers giving an accuracy of about 95% in predicting Alzheimer's disease.

A biomarker panel may comprise or consist of TUBB and one additional biomarker selected from Table 2.

A biomarker panel may comprise or consist of TUBB and two additional biomarkers selected from Table 2.

A biomarker panel may comprise or consist of TUBB and three additional biomarkers selected from Table 2.

A biomarker panel may comprise or consist of TUBB and four additional biomarkers selected from Table 2.

A biomarker panel may comprise or consist of TUBB and five additional biomarkers selected from Table 2. This biomarker panel may provide about 95% accuracy in predicting Alzheimer's disease.

Present disclosure relates to the use of a biomarker panel comprising TUBB as a biomarker for Alzheimer's disease, wherein expression of TUBB is decreased in a sample from a subject with Alzheimer's disease compared to a comparable sample from a healthy individual.

Present disclosure relates to the use of a biomarker panel comprising NEAT1 as a biomarker for Alzheimer's disease, wherein expression of NEAT1 is decreased in a sample from a subject with Alzheimer's disease compared to a comparable sample from a healthy individual.

Present disclosure relates to the use of a biomarker panel comprising CENPB as a biomarker for Alzheimer's disease, wherein expression of CENPB is decreased in a sample from a subject with Alzheimer's disease compared to a comparable sample from a healthy individual.

Present disclosure relates to the use of a biomarker panel comprising AA482221 as a biomarker for Alzheimer's diesease, wherein expression of AA482221 is decreased in a sample from a subject with Alzheimer's disease compared to a comparable sample from a healthy individual.

Present disclosure relates to the use of a biomarker panel comprising CPSF3 as a biomarker for Alzheimer's disease, wherein expression of CPSF3 is decreased in a sample from a subject with Alzheimer's disease compared to a comparable sample from a healthy individual.

Present disclosure relates to the use of a biomarker panel comprising seryl-tRNA synthetase for detection of Alzheimer's disease.

Present disclosure relates to the use of a biomarker panel comprising stathmin-like 2 for detection of Alzheimer's disease.

Present disclosure relates to the use of a biomarker panel comprising RNA binding motif protein, X-linked for detection of Alzheimer's disease, or Use of a biomarker panel comprising anaphase promoting complex subunit 13 for detection of Alzheimer's disease, or Use of a biomarker panel comprising reticulon 3 for detection of Alzheimer's disease, or Use of a biomarker panel comprising proteasome (prosome, macropain) subunit, beta type, 3 for detection of Alzheimer's disease, or Use of a biomarker panel comprising hypothetical LOC100509179 for detection of Alzheimer's disease, or Use of a biomarker panel comprising optineurin for detection of Alzheimer's disease, or Use of a biomarker panel comprising tyrosine 3-monooxygenase/tryptophan 5-monooxygenase activation protein, for detection of Alzheimer's disease, or Use of a biomarker panel comprising zeta polypeptide for detection of Alzheimer's disease, or Use of a biomarker panel comprising OCIA domain containing 1 for detection of Alzheimer's disease, or Use of a biomarker panel comprising ATPase, H+ transporting, lysosomal 31kDa, V1 subunit E1 for detection of Alzheimer's disease, or Use of a biomarker panel comprising fibroblast growth factor (acidic) intracellular binding protein for detection of Alzheimer's disease, or Use of a biomarker panel comprising aftiphilin for detection of Alzheimer's disease, or Use of a biomarker panel comprising Berardinelli-Seip congenital lipodystrophy 2 (seipin) for detection of Alzheimer's disease, or Use of a biomarker panel comprising glutamic-oxaloacetic transaminase 1, soluble (aspartate aminotransferase 1) for detection of Alzheimer's disease, or Use of a biomarker panel comprising ATPase, Ca+ + transporting, cardiac muscle, slow twitch 2 for detection of Alzheimer's disease, or Use of a biomarker panel comprising DAZ interacting protein 3, zinc finger for detection of Alzheimer's disease, or Use of a biomarker panel comprising CAP, adenylate cyclase-associated protein 1 for detection of Alzheimer's disease, or Use of a biomarker panel comprising tubulin, alpha 1b for detection of Alzheimer's disease, or Use of a biomarker panel comprising NADH dehydrogenase (ubiquinone) 1 alpha subcomplex, 7, 14.5kDa for detection of Alzheimer's disease, or Use of a biomarker panel comprising eukaryotic translation initiation factor 2B, subunit 3 gamma, 58kDa for detection of Alzheimer's disease, or Use of a biomarker panel comprising Use of a biomarker panel comprising ribosomal protein L15 for detection of Alzheimer's disease, or Use of a biomarker panel comprising tubulin, alpha 1b for detection of Alzheimer's disease, or Use of a biomarker panel comprising proteasome (prosome, macropain) 26S subunit, non-ATPase, 1 for detection of Alzheimer's disease, or Use of a biomarker panel comprising proteasome (prosome, macropain) subunit, beta type, 2 for detection of Alzheimer's disease, or Use of a biomarker panel comprising ATP synthase, H+ transporting, mitochondrial F1 complex, gamma polypeptide 1 for detection of Alzheimer's disease, or Use of a biomarker panel comprising tetratricopeptide repeat domain 15 for detection of Alzheimer's disease, or Use of a biomarker panel comprising processing of precursor 4, ribonuclease P/MRP subunit (S. cerevisiae) for detection of Alzheimer's disease, or Use of a biomarker panel comprising rabaptin, RAB GTPase binding effector protein 1 for detection of Alzheimer's disease.

Where a sample from a subject shows only a slight increase or a decrease in the amount of one or more biomarkers the subject may be classified as having mild cognitive impairment (MCI) rather than Alzheimer's disease.

The biomarkers disclosed may be used as one or more of diagnostic biomarkers, prognostic biomarkers and therapeutic biomarkers. Biomarkers which are not explicitly stated to be therapeutic biomarkers are preferably at least one of diagnostic or prognostic biomarkers, or both.

Preferably the method allows the diagnosis of Alzheimer's disease in a subject from the analysis of the level of the one or more biomarkers in a sample provided by/or obtained from the subject.

The method disclosed may also or alternatively be used to develop therapies targeted to the biomarkers. Reference herein to therapeutic biomarkers is intended to refer to biomarkers which can be used as targets for therapy, this may be in addition to or an alternative to the biomarkers being used for diagnostic and/or prognostic purposes. Preferably a therapeutic biomarker represents a protein which can be targeted by a ligand to deliver a drug, for example, the therapeutic biomarker may be recognised by an antibody-drug conjugate wherein the antibody recognises the therapeutic biomarker and delivers the conjugated drug.

If the expression level of one or more of the genes selected from Table 1 and optionally Table 2 is elevated or decreased relative to the standard this indicates that the subject may have Alzheimer's disease.

If the expression level of one or more genes from Table 1 and optionally Table 2 in a sample from an individual is found to be higher or lower than expected this may indicate that the indicate that the individual may have Alzheimer's disease. The individual may be selected for treatment for Alzheimer's disease and the individual may be treated for Alzheimer's disease.

The method may be used in one or more of the following; diagnosing whether or not a subject has Alzheimer's disease; advising on the prognosis for a subject with Alzheimer's disease and monitoring the effectiveness or response of a subject to a particular treatment for Alzheimer's disease.

At least one of the genes from Table 1 and optionally at least one of the genes from Table 2 may be used as a biomarker panel where the expression level of genes is measured to distinguish between subjects having Alzheimer's disease and healthy subjects with a high degree of accuracy. The accuracy is increased as the number of biomarkers increases. This biomarker panel is advantageous because it provides a high degree of accuracy with only a small number of biomarkers.

The altered expression level is an expression level that is higher or lower than the expression level expected in a subject not having Alzheimer's disease.

The expression level of the gene may be determined using the level of mRNA encoded by the selected gene present in the sample.

The expression level of the gene may be determined using quantitative PCR.

The expression level of the gene may be determined using the level of protein encoded by the selected gene present in the sample.

The expression level of one or more further genes may also be determined.

In another aspect the present invention provides a kit for use in determining the Alzheimer's disease status in a subject comprising at least one agent specific determining the expression level of TUBB, NEAT1, CENPB, AA482221 and CPSF3 in a sample from a subject and instructions for determining the Alzheimer's disease status of the subject.

The kit may further comprise at least one agent for determining the expression level of one or more genes listed in Table 2.

The agent may be an oligonucleotide.

In a further aspect the present invention provides use of the determination of the expression level of: TUBB, NEAT1, CENPB, AA482221 and CPSF3 and optionally one or more genes from Table 2 as a means to determine the Alzheimer's disease status of a subject.

The present disclosure further relates to a gene expression product from a gene selected from the group consisting of TUBB, NEAT1, CENPB, AA482221 and CPSF3 or the expression product from a gene listed in Table 2 for use as biomarker for subjects having Alzheimer's disease.

The present disclosure further relates to an oligonucleotide capable of detecting the presence or expression level of a gene expression product from a gene selected from the group consisting of TUBB, NEAT1, CENPB, AA482221 and CPSF3 or a gene selected from the genes listed in Table 2 in a sample from a subject.

Disclosed is a method, kit, use, gene or oligonucleotide as described herein wherein the sample is a sample of blood, serum, cerebrospinal fluid, saliva, urine, tissue fluid, tissue, brain tissue.

The sample of material may be a sample of blood, sputum, saliva, wound exudate, urine, faeces, peritoneal fluid or any respiratory secretion. A sample of blood may be whole blood, blood plasma or blood serum.

The sample may be a sample of whole blood or a fraction of whole blood. Blood samples have the advantage that they are readily obtainable and tend to be more homogenous in nature than other sample types. Samples of whole blood contain RNA which can be extracted to generate a transcriptional profile.

The sample may be a sample of cerebrospinal fluid.

Where the sample is a sample of blood, whole blood, a fraction of whole blood or a sample of cerebrospinal fluid the biomarkers may be protein products of the genes listed in Table 1 or Table 2.

The methods disclosed are advantageous as they permit both the regular screening of susceptible populations and also the testing of all individuals who are suspected of having Alzheimer's disease, and thus enables more timely treatment possibly preventing or reducing the impact of symptoms. It also permits earlier diagnosis than is available using conventional diagnosis techniques and therefore Alzheimer's disease can be treated earlier. It also permits a quantitative assessment of the efficacy of therapy resulting in interventions that are customised to the response of the individual patient. It also provides a tool for widespread use in epidemiological studies, an important consideration as Alzheimer's disease becomes more prevalent.

The genes listed in Table 1 and Table 2 may be targets for development of new therapeutics for treating Alzheimer's disease.

The skilled man will appreciate that preferred features of any one embodiment and/or aspect of the invention may be applied to all other embodiments or aspects of the invention.

The present invention will be further described in more detail, by way of example only, with reference to the following Figures in which:

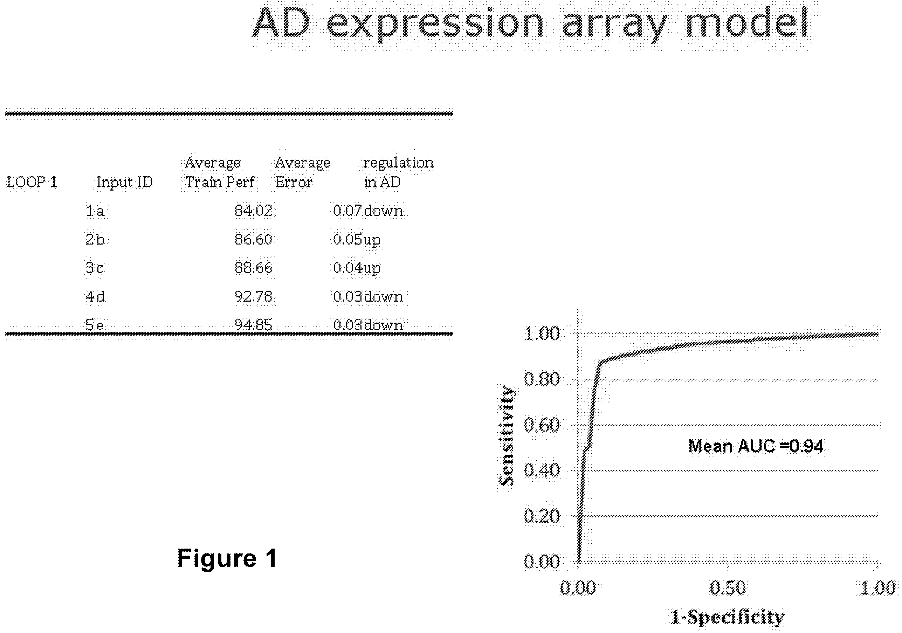

**Figure 1** shows data from an Alzheimer's disease expression array model;
**Figure 2** shows data from an Alzheimer's disease expression array model;
**Figure 3** shows the population distribution for predicted groups from 5 marker panel model;

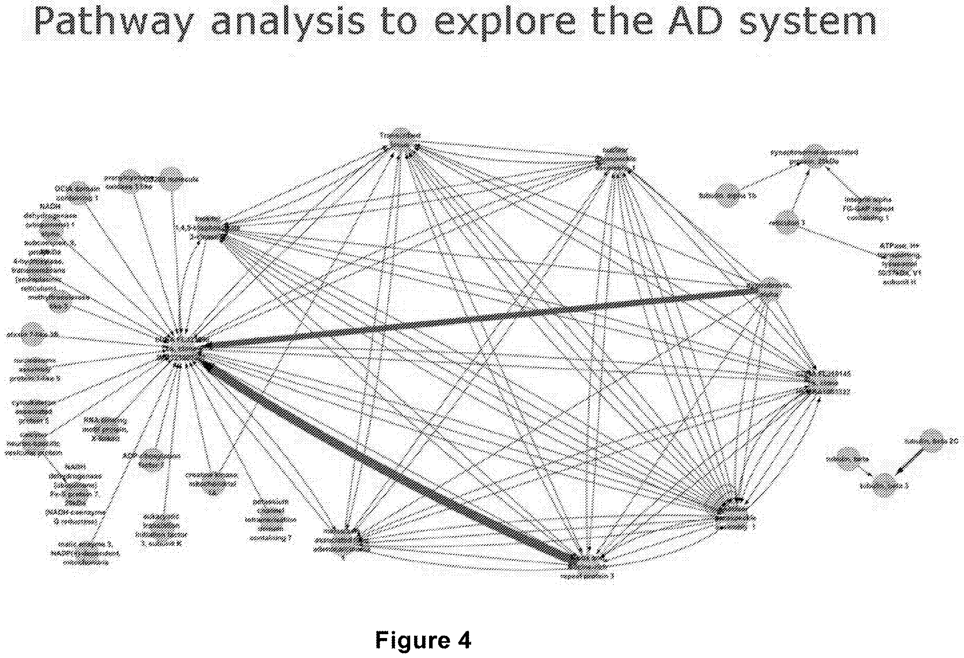

**Figure 4** shows a pathway analysis to explore the Alzheimer's disease system; and
**Figure 5** shows the distribution of performance of single genes as biomarkers for predicting Alzheimer's disease.

Raw data used to produce the gene panels was taken from Liang et al., Physiol Genomics, 2007, February 12; 28(3): 311-322.

In Liang et al data was obtained from Tissue samples (200 *µ*m of sectioned tissue) from brain regions histopathologically or metabolically relevant to AD. Gene expression profiles from laser capture microdissected neurons in six functionally and anatomically distinct regions from clinically and histopathologically normal aged human brains were produced. These regions, which are also known to be differentially vulnerable to the histopathological and metabolic features of Alzheimer's disease (AD), they include the entorhinal cortex and hippocampus (limbic and paralimbic areas vulnerable to early neurofibrillary tangle pathology in AD), posterior cingulate cortex (a paralimbic area vulnerable to early metabolic abnormalities in AD), temporal and prefrontal cortex (unimodal and heteromodal sensory association areas vulnerable to early neuritic plaque pathology in AD), and primary visual cortex (a primary sensory area relatively spared in early AD). These neuronal profiles will provide valuable reference information for future studies of the brain, in normal aging, AD and other neurological and psychiatric disorders.

Presence of AD was confirmed at post mortem for each of the brains that AD tissue was obtained from.

Plaque regions were histologically identified for each brain and isolated by LCM.

Profiled using Affymetrix U133 Plus 2.0 array

N = 89 AD, 74 controls (age matched), 34 MCI - BY MMSE (blind samples)

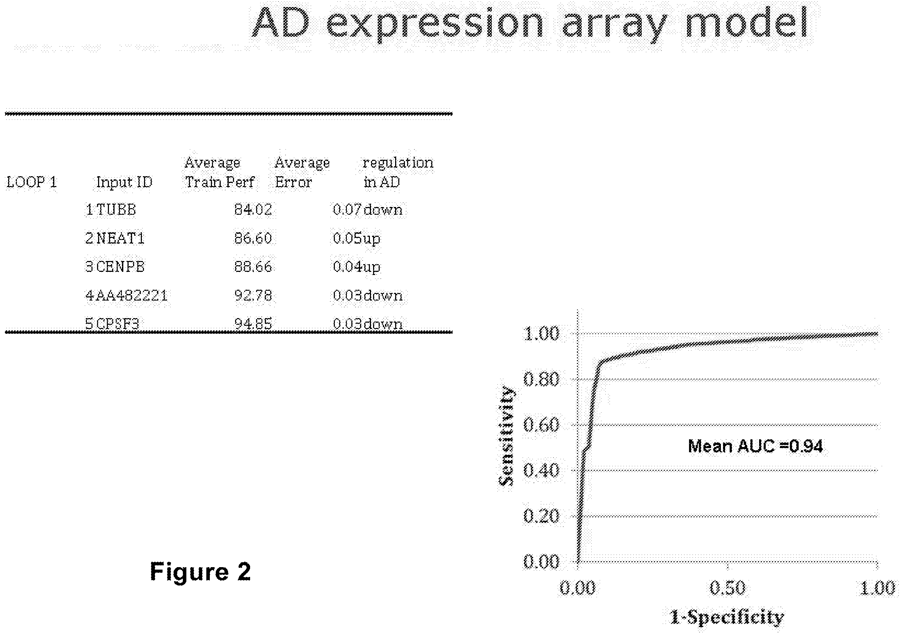

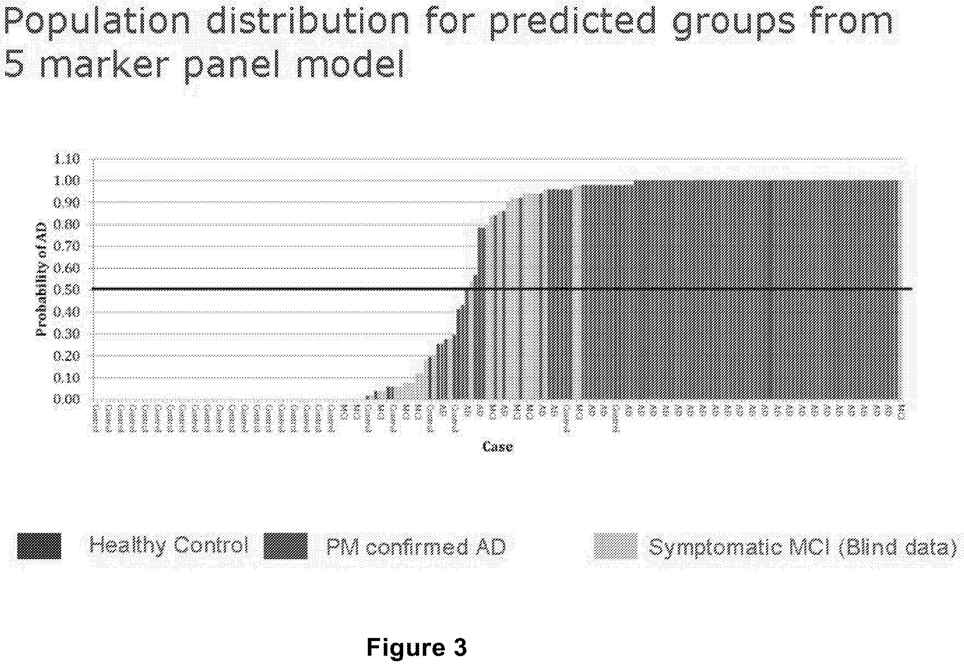

The data was analysed using a data mining algorithm and method both described in patent application number PCT/GB2009/051412, published as WO2010/046697 and claiming priority to GB 0819221.3. This method provided two panels of biomarkers.
1. Table 1 is a panel of biomarkers which were used together in a diagnostic model. All 5 of these markers were used together and separately to predict whether a subject has Alzheimer's disease. The accuracy of the prediction was calculated and the results shown in figure 2. In Figure 2 it is shown whether each biomarker is up regulated or down regulated in samples from subjects with Alzheimer's disease compared to samples from healthy subjects (right hand column of figure 3). The cumulative percentage accuracy is shown in the 3^{rd} column in the Table in figure 2, for example, TUBB is down regulated in samples from subjects with Alzheimer's disease compared to samples from healthy subjects. The accuracy of prediction of Alzheimer's disease with TUBB alone was calculated as 84.02 %. The Accuracy of prediction of Alzheimer's disease increased with each additional biomarker as shown in the Table in Figure 2 where the percentage accuracy is shown in the column marked "Average Train Perf" and the percentage accuracy is cumulative going down that column.
2. The panel of biomarkers in Table 2 are single markers which are predictive on their own but may constitute an improved signature or alternative solution when added to those in Table 1.

## Claims

1. A method of providing an indication useful in distinguishing between healthy subjects and subjects having Alzheimer's disease comprising:
(a) providing a sample of material which was obtained from the subject; and
(b) determining the expression level of the TUBB, NEAT1, CENPB, AA482221 and CPSF3 genes in the sample;
wherein the expression level determined in (b) provides an indication useful in distinguishing between healthy subjects and subjects having Alzheimer's disease.

2. The method of claim 1 further comprising:
(c) determining the expression level of one or more genes selected from the group consisting of:
tubulin, alpha 1c
seryl-tRNA synthetase
stathmin-like 2
RNA binding motif protein, X-linked
anaphase promoting complex subunit 13
reticulon 3
proteasome (prosome, macropain) subunit, beta type, 3
hypothetical LOC100509179
optineurin
tyrosine 3-monooxygenase/tryptophan 5-monooxygenase activation protein, zeta polypeptide OCIA domain containing 1
ATPase H+ transporting, lysosomal 31kDa V1 subunit E1
fibroblast growth factor (acidic) intracellular binding protein
aftiphilin
Berardinelli-Seip congenital lipodystrophy 2 (seipin)
glutamic-oxaloacetic transaminase 1, soluble (aspartate aminotransferase 1)
ATPase Ca++ transporting, cardiac muscle, slow twitch 2
DAZ interacting protein 3, zinc finger
CAP, adenylate cyclase-associated protein 1
NADH dehydrogenase (ubiquinone) 1 alpha subcomplex, 7, 14.5kDa
eukaryotic translation initiation factor 2B, subunit 3 gamma, 58kDa
ribosomal protein L15
tubulin, alpha 1b
proteasome (prosome, macropain) 26S subunit, non-ATPase, 1
proteasome (prosome, macropain) subunit, beta type, 2
ATP synthase, H+ transporting, mitochondrial F1 complex, gamma polypeptide 1 tetratricopeptide repeat domain 15
processing of precursor 4, ribonuclease P/MRP subunit (S. cerevisiae) rabaptin, RAB GTPase binding effector protein 1.

3. The method of claim 1 or claim 2 further comprising:
(d) employing the expression level determined in (b) and optionally (c) to provide an indication useful in distinguishing between healthy subjects and subjects having Alzheimer's disease.

4. The method of claim 3 wherein the expression level determined in (b) and optionally (c) is compared to a standard, wherein the standard may optionally be the expression level that would be expected in a healthy individual.

5. The method according to claim 4 wherein if the expression level of TUBB, NEAT1, CENPB, AA482221 and CPSF3 is elevated or decreased relative to the standard this indicates that the subject may have Alzheimer's disease.

6. The method of claim 4 or 5 wherein a subject suspected of having Alzheimer's disease is selected for treatment.

7. The method of any one of the preceding claims wherein the method is for further use in one or more of the following; advising on the prognosis for a subject suspected of having Alzheimer's disease and monitoring the effectiveness or response of a subject to a particular treatment for Alzheimer's disease.

8. A method according to any one of the preceding claims wherein the expression level of the gene is determined using the level of mRNA encoded by the selected gene present in the sample.

9. The method according to claim 8 wherein the level of mRNA in the sample is determined using quantitative PCR, analysis of expression arrays, next generation sequencing, comparative genomic hybridisation arrays, or multiplexed PCR.

10. A method according to any one of the preceding claims wherein the expression level of the gene is determined using the level of protein encoded by the selected gene present in the sample.

11. The method according to claim 10 wherein the level of protein in the sample is determined by ELISA, immunohistochemistry, protein aptamer arrays or protein immunological arrays.

12. A method according to any one of the preceding claims wherein the expression level of one or more further genes is also determined.

13. A kit consisting of:
an agent specific for determining the expression level of TUBB;
an agent specific for determining the expression level of NEAT1;
an agent specific for determining the expression level of CENPB;
an agent specific for determining the expression level of AA482221;
an agent specific for determining the expression level CPSF3; and
instructions for determining the Alzheimer's disease status of the subject.

14. A kit consisting of:
an agent specific for determining the expression level of TUBB;
an agent specific for determining the expression level of NEAT1;
an agent specific for determining the expression level of CENPB;
an agent specific for determining the expression level of AA482221;
an agent specific for determining the expression level CPSF3; and
an agent specific for determining the expression level of one or more of the following genes:
tubulin, alpha 1c
seryl-tRNA synthetase
stathmin-like 2
RNA binding motif protein, X-linked
anaphase promoting complex subunit 13
reticulon 3
proteasome (prosome, macropain) subunit, beta type, 3
hypothetical LOC100509179
optineurin
tyrosine 3-monooxygenase/tryptophan 5-monooxygenase activation protein, zeta polypeptide OCIA domain containing 1
ATPase H+ transporting, lysosomal 31kDa V1 subunit E1
fibroblast growth factor (acidic) intracellular binding protein
aftiphilin
Berardinelli-Seip congenital lipodystrophy 2 (seipin)
glutamic-oxaloacetic transaminase 1, soluble (aspartate aminotransferase 1)
ATPase Ca++ transporting, cardiac muscle, slow twitch 2
DAZ interacting protein 3, zinc finger
CAP, adenylate cyclase-associated protein 1
NADH dehydrogenase (ubiquinone) 1 alpha subcomplex, 7, 14.5kDa
eukaryotic translation initiation factor 2B, subunit 3 gamma, 58kDa
ribosomal protein L15
tubulin, alpha 1b
proteasome (prosome, macropain) 26S subunit, non-ATPase, 1
proteasome (prosome, macropain) subunit, beta type, 2
ATP synthase, H+ transporting, mitochondrial F1 complex, gamma polypeptide 1 tetratricopeptide repeat domain 15
processing of precursor 4, ribonuclease P/MRP subunit (S. cerevisiae) rabaptin, RAB GTPase binding effector protein 1;
and
instructions for determining the Alzheimer's disease status of the subject

15. Use of a method wherein the expression level of TUBB, NEAT1, CENPB, AA482221 and CPSF3 is determined, wherein said expression levels are used as a means of providing an indication useful in distinguishing between healthy subjects and subjects having Alzheimer's disease.

16. The use according to claim 15 wherein the expression level of one or more genes listed in claim 2 is also determined to providing an indication useful in distinguishing between healthy subjects and subjects having Alzheimer's disease.

17. A method or use according to any one of the preceding claims wherein the sample is a sample of blood, serum, cerebrospinal fluid, saliva, urine, tissue fluid, tissue, brain tissue.

18. A method of selecting a subject for treatment for Alzheimer's disease or for further investigation of Alzheimer's disease comprising the steps of:
i) determining the amount of expression of TUBB, NEAT1, CENPB, AA482221 and CPSF3, and optionally one or more of the biomarkers listed in claim 2, in a sample from the subject;
ii) determining if the amount of expression of each of TUBB, NEAT1, CENPB, AA482221 and CPSF3 is elevated or decreased relative to the expected level of expression of that gene in a comparable sample from a healthy subject,
wherein the subject is selected for treatment for Alzheimer's disease or for further investigation of Alzheimer's disease if the subject is determined to have elevated or reduced amounts of TUBB, NEAT1, CENPB, AA482221 and CPSF3.

## Patentansprüche

1. Verfahren zum Bereitstellen einer Angabe, die zum Unterscheiden zwischen gesunden Subjekten und Subjekten mit Alzheimer-Krankheit verwendet werden kann, Folgendes umfassend:
(a) Bereitstellen einer Probe von Material, das von einem Subjekt erhalten wurde; und
(b) Bestimmen des Expressionsniveaus des TUBB-, NEAT1-, CENPB-, AA482221- und CPSF3-Gens in der Probe;
wobei das in (b) bestimmte Expressionsniveau eine Angabe bereitstellt, die zum Unterscheiden zwischen gesunden Subjekten und Subjekten mit Alzheimer-Krankheit verwendet werden kann.

2. Verfahren nach Anspruch 1, ferner umfassend:
(c) Bestimmen des Expressionsniveaus eines oder mehrerer Gene, ausgewählt aus der Gruppe, die aus Folgendem besteht:
Tubulin, alpha 1c
Seryl-tRNA-Synthetase
Stathmin-ähnlichem Protein 2
RNA-bindendem Motivprotein, X-gebunden
Untereinheit 13 eines Anaphase-fördernden Komplexes
Reticulon 3
Untereinheit von Proteasom (Prosom, Macropain), Beta-Typ, 3
hypothetischem LOC100509179
Optineurin
Tyrosin-3-Monooxygenase/Tryptophan-5-Monooxygenase-Aktivierungsprotein, Zeta-Polypeptid OCIA-Domäne-enthaltendem Protein 1
ATPase, H⁺-transportierend, lysosomale 31kDa-V1-Untereinheit E1
Fibroblastenwachstumsfaktor (säurehaltig) intrazellulär bindendem Protein Aftiphilin
Berardinelli-Seip kongenitaler Lipodystrophie 2 (Seipin)
Glutamat-Oxalacetat-Transaminase 1, löslich (Aspartat-Aminotransferase 1)
ATPase, Ca⁺⁺-transportierend, Herzmuskel, langsam zuckend, 2
mit DAZ interagierendem Protein 3, Zinkfinger
CAP-, Adenylatcyclase-assoziiertem Protein 1
1-alpha-Subkomplex von NADH-Dehydrogenase (Ubichinon), 7, 14,5 kDa eukaryotischer Translations-Initiationsfaktor 2B, Untereinheit 3 Gamma, 58 kDa ribosomalem Protein L15
Tubulin, alpha 1b
26S-Untereinheit von Proteasom (Prosom, Macropain), nicht-ATPase, 1 Untereinheit von Proteasom (Prosom, Macropain), Beta-Typ, 2
ATP-Synthase, H⁺-transportierend, mitochondrialer F1-Komplex, Gamma-Polypeptid 1 Tetratricopeptid-Repetierdomäne 15
Verarbeitung von Precursor 4, Untereinheit von Ribonuklease P/MRP (S. cerevisiae) Rabaptin,
RAB-GTPase-bindendem Effektorprotein 1.

3. Verfahren nach Anspruch 1 oder 2, ferner umfassend:
(d) Anwenden des in (b) und optional des in (c) bestimmten Expressionsniveaus zum Bereitstellen einer Angabe, die zum Unterscheiden zwischen gesunden Subjekten und Subjekten mit der Alzheimer-Krankheit verwendet werden kann.

4. Verfahren nach Anspruch 3, wobei das in (b) und optional das in (c) bestimmte Expressionsniveau mit einem Standard verglichen wird, wobei der Standard optional das Expressionsniveau ist, das bei einem gesunden Individuum erwartet werden würde.

5. Verfahren nach Anspruch 4, wobei, wenn das Expressionsniveau von TUBB, NEAT1, CENPB, AA482221 und CPSF3 im Verhältnis zu dem Standard erhöht oder verringert ist, dies angibt, dass das Subjekt an der Alzheimer-Krankheit leiden könnte.

6. Verfahren nach Anspruch 4 oder 5, wobei ein Subjekt, bei dem die Alzheimer-Krankheit vermutet wird, für die Behandlung ausgewählt wird.

7. Verfahren nach einem der vorhergehenden Ansprüche, wobei das Verfahren ferner für eines oder mehrere der Folgenden verwendet werden kann; Beraten bezüglich der Prognose für ein Subjekt, bei dem die Alzheimer-Krankheit vermutet wird, und Überwachen der Effektivität oder der Reaktion eines Subjekts auf eine jeweilige Behandlung der Alzheimer-Krankheit.

8. Verfahren nach einem der vorhergehenden Ansprüche, wobei das Expressionsniveau des Gens unter Verwendung des Niveaus von mRNA, die durch das ausgewählte, in der Probe enthaltene Gen kodiert ist, bestimmt wird.

9. Verfahren nach Anspruch 8, wobei das Niveau von mRNA in der Probe unter Verwendung von quantitativer PCR, einer Analyse von Expressionsarrays, einer Sequenzierung der nächsten Generation, eines komparativen genomischen Hybridisierungs-Arrays oder einer Multiplex-PCR bestimmt wird.

10. Verfahren nach einem der vorhergehenden Ansprüche, wobei das Expressionsniveau des Gens unter Verwendung des Niveaus des Proteins, das durch das ausgewählte, in der Probe enthaltene Gen kodiert ist, bestimmt wird.

11. Verfahren nach Anspruch 10, wobei das Niveau des Proteins in der Probe durch ELISA, Immunhistochemie, Protein-Aptamer-Arrays oder immunologische Protein-Arrays bestimmt wird.

12. Verfahren nach einem der vorhergehenden Ansprüche, wobei auch das Expressionsniveau von einem oder von mehreren weiteren Genen bestimmt wird.

13. Kit, bestehend aus:
einem Agens, das spezifisch ist für das Bestimmen des Expressionsniveaus von TUBB;
einem Agens, das spezifisch ist für das Bestimmen des Expressionsniveaus von NEAT1;
einem Agens, das spezifisch ist für das Bestimmen des Expressionsniveaus von CENPB;
einem Agens, das spezifisch ist für das Bestimmen des Expressionsniveaus von AA482221;
einem Agens, das spezifisch ist für das Bestimmen des Expressionsniveaus von CPSF3; und
Anweisungen zum Bestimmen des Alzheimer-Krankheitszustandes des Subjekts.

14. Kit, bestehend aus:
einem Agens, das spezifisch ist für das Bestimmen des Expressionsniveaus von TUBB;
einem Agens, das spezifisch ist für das Bestimmen des Expressionsniveaus von NEAT1;
einem Agens, das spezifisch ist für das Bestimmen des Expressionsniveaus von CENPB;
einem Agens, das spezifisch ist für das Bestimmen des Expressionsniveaus von AA482221;
einem Agens, das spezifisch ist für das Bestimmen des Expressionsniveaus von CPSF3; und
einem Agens, das spezifisch ist für das Bestimmen des Expressionsniveaus von einem oder mehreren der folgenden Gene:
Tubulin, alpha 1c
Seryl-tRNA-Synthetase
Stathmin-ähnlichem Protein 2
RNA-bindendem Motivprotein, X-gebunden
Untereinheit 13 eines Anaphase-fördernden Komplexes
Reticulon 3
Untereinheit von Proteasom (Prosom, Macropain), Beta-Typ, 3
hypothetischem LOC100509179
Optineurin
Tyrosin-3-Monooxygenase/Tryptophan-5-Monooxygenase-Aktivierungsprotein, Zeta-Polypeptid OCIA-Domäne-enthaltendem Protein 1
ATPase, H⁺-transportierend, lysosomale 31kDa-V1-Untereinheit E1
Fibroblastenwachstumsfaktor (säurehaltig) intrazellulär bindendem Protein
Aftiphilin
Berardinelli-Seip kongenitaler Lipodystrophie 2 (Seipin)
Glutamat-Oxalacetat-Transaminase 1, löslich (Aspartat-Aminotransferase 1)
ATPase, Ca⁺⁺-transportierend, Herzmuskel, langsam zuckend, 2
mit DAZ interagierendem Protein 3, Zinkfinger
CAP-, Adenylatcyclase-assoziiertem Protein 1
1-alpha-Subkomplex von NADH-Dehydrogenase (Ubichinon), 7, 14,5 kDa eukaryotischem Translations-Initiationsfaktor 2B, Untereinheit 3 Gamma, 58 kDa ribosomalem Protein L15
Tubulin, alpha 1b
26S-Untereinheit von Proteasom (Prosom, Macropain), nicht-ATPase, 1 Untereinheit von Proteasom (Prosom, Macropain), Beta-Typ, 2
ATP-Synthase, H⁺-transportierend, mitochondrialer F1-Komplex, Gamma-Polypeptid 1 Tetratricopeptid-Repetierdomäne 15
Verarbeitung von Precursor 4, Untereinheit von Ribonuklease P/MRP (S. cerevisiae) Rabaptin, RAB-GTPase-bindendem Effektorprotein 1;
und
Anweisungen zum Bestimmen des Alzheimer-Krankheitszustandes des Subjekts.

15. Verwendung eines Verfahrens, wobei das Expressionsniveau von TUBB, NEAT1, CENPB, AA482221 und CPSF3 bestimmt wird, wobei die Expressionsniveaus als Mittel zum Bereitstellen einer Angabe verwendet werden, die zum Unterscheiden zwischen gesunden Subjekten und Subjekten mit Alzheimer-Krankheit verwendet werden kann.

16. Verwendung nach Anspruch 15, wobei das Expressionsniveau von einem oder mehreren in Anspruch 2 aufgelisteten Genen auch bestimmt wird, um eine Angabe bereitzustellen, die zum Unterscheiden zwischen gesunden Subjekten und Subjekten mit Alzheimer-Krankheit verwendet werden kann.

17. Verfahren oder Verwendung nach einem der vorhergehenden Ansprüche, wobei die Probe eine Probe von Blut, Serum, Cerebrospinalflüssigkeit, Speichel, Urin, Gewebeflüssigkeit, Gewebe, Hirngewebe ist.

18. Verfahren zum Auswählen eines Subjekts zum Behandeln der Alzheimer-Krankheit oder zum weiteren Untersuchen der Alzheimer-Krankheit, folgende Schritte umfassend:
i) Bestimmen der Expressionsstärke von TUBB, NEAT1, CENPB, AA482221 und CPSF3 und optional eines oder mehrerer der in Anspruch 2 aufgelisteten Biomarker, in einer Probe von dem Subjekt;
ii) Bestimmen, ob die Expressionsstärke jedes von TUBB, NEAT1, CENPB, AA482221 und CPSF3 im Verhältnis zu dem erwarteten Expressionsniveau jenes Gens in einer vergleichbaren Probe von einem gesunden Subjekt erhöht oder verringert ist,
wobei das Subjekt zum Behandeln der Alzheimer-Krankheit oder zum weiteren Untersuchen der Alzheimer-Krankheit ausgewählt wird, wenn bei dem Subjekt erhöhte oder verringerte Stärken von TUBB, NEAT1, CENPB, AA482221 und CPSF3 bestimmt werden.

## Revendications

1. Procédé de fourniture d'une indication utile dans la distinction entre les sujets sains et les sujets atteints de la maladie d'Alzheimer, comprenant :
(a) la fourniture d'un échantillon de matériau qui a été obtenu du sujet ; et
(b) la détermination du niveau d'expression des gènes TUBB, NEAT1, CENPB, AA482221 et CPSF3 dans l'échantillon ;
le niveau d'expression déterminé en (b) fournissant une indication utile dans la distinction entre les sujets sains et les sujets atteints de la maladie d'Alzheimer.

2. Procédé selon la revendication 1 comprenant en outre :
(c) la détermination du niveau d'expression d'un ou de plusieurs gène(s) choisi(s) dans le groupe constitué par :
tubuline, alpha1c
séryl-ARNt synthétase
stathmine-type 2
protéine du motif de fixation à l'ARN, liée à X
sous-unité 13 du complexe favorisant l'anaphase
réticulon 3
sous-unité du protéasome (prosome, macropaïne), type bêta, 3
LOC100509179 hypothétique
optineurine
protéine d'activation de tyrosine 3-monooxygénase/tryptophane 5-monooxygénase, polypeptide zêta
protéine 1 contenant le domaine OCIA
ATPase de transport de H+, sous-unité lysosomale E1 du V1 de 31 kDa
protéine de liaison intracellulaire (acide) du facteur de croissance des fibroblastes aftiphiline
protéine 2 de la lipodystrophie congénitale de Berardinelli-Seip (séipine) transaminase glutamique oxalo-acétique 1, soluble (aspartate aminotransférase 1)
ATPase de transport de Ca++, muscle cardiaque, contraction lente de type 2 protéine 3 d'interaction de DAZ, en doigt de zinc
CAP, protéine 1 associée à l'adénylate cyclase
sous-complexe alpha de la NADH déhydrogénase (ubiquinone) 1, 7, 14,5 kDa
facteur 2B d'initiation de la traduction eucaryote, sous-unité gamma 3, 58 kDa
protéine ribosomique L15
tubuline, alpha 1b
sous-unité 26S du protéasome (prosome, macropaïne), non-ATPase, 1
sous-unité du protéasome (prosome, macropaïne), type bêta, 2
ATP synthase, de transport de H+, complexe F1 mitochondrial, polypeptide gamma 1 domaine 15 des répétitions tétratricopeptides
transformation du précurseur 4, sous-unité de la ribonucléase P/MRP (S. cerevisiae) rabaptine, RAB GTPase
protéine 1 effectrice de liaison.

3. Procédé selon la revendication 1 ou 2 comprenant en outre :
(d) l'emploi du niveau d'expression déterminé en (b) et facultativement en (c) pour fournir une indication utile dans la distinction entre les sujets sains et les sujets atteints de la maladie d'Alzheimer.

4. Procédé selon la revendication 3, dans lequel le niveau d'expression déterminé en (b) et facultativement en (c) est comparé à un étalon, l'étalon pouvant être facultativement le niveau d'expression qui serait prévu chez un individu sain.

5. Procédé selon la revendication 4, dans lequel, si le niveau d'expression de TUBB, NEAT1, CENPB, AA482221 et CPSF3 est élevé ou réduit par rapport à l'étalon, ceci indique que le sujet peut être atteint de la maladie d'Alzheimer.

6. Procédé selon la revendication 4 ou 5, dans lequel un sujet suspecté d'être atteint de la maladie d'Alzheimer est sélectionné pour le traitement.

7. Procédé selon l'une quelconque des revendications précédentes, le procédé étant également utilisable dans un ou plusieurs des cas suivants ; fournir un avis sur le pronostic pour un sujet suspecté d'être atteint de la maladie d'Alzheimer et contrôler l'efficacité ou la réponse d'un sujet à un traitement particulier de la maladie d'Alzheimer.

8. Procédé selon l'une quelconque des revendications précédentes, dans lequel le niveau d'expression du gène est déterminé en utilisant le niveau d'ARNm codé par le gène choisi présent dans l'échantillon.

9. Procédé selon la revendication 8, dans lequel le niveau d'ARNm dans l'échantillon est déterminé en utilisant la PCR quantitative, l'analyse des matrices d'expression, le séquençage de nouvelle génération, les matrices d'hybridation génomique comparative ou la PCR multiplexe.

10. Procédé selon l'une quelconque des revendications précédentes, dans lequel le niveau d'expression du gène est déterminé en utilisant le niveau de protéine codé par le gène choisi présent dans l'échantillon.

11. Procédé selon la revendication 10, dans lequel le niveau de protéine dans l'échantillon est déterminé par ELISA, immunohistochimie, matrices d'aptamères de protéines ou matrices immunologiques de protéines.

12. Procédé selon l'une quelconque des revendications précédentes, dans lequel le niveau d'expression d'un ou de plusieurs gène(s) est également déterminé.

13. Kit comprenant :
un agent spécifique pour déterminer le niveau d'expression de TUBB ;
un agent spécifique pour déterminer le niveau d'expression de NEAT1 ;
un agent spécifique pour déterminer le niveau d'expression de CENPB ;
un agent spécifique pour déterminer le niveau d'expression de AA482221 ;
un agent spécifique pour déterminer le niveau d'expression de CPSF3 ; et
des instructions pour déterminer l'état de la maladie d'Alzheimer du sujet.

14. Kit comprenant :
un agent spécifique pour déterminer le niveau d'expression de TUBB ;
un agent spécifique pour déterminer le niveau d'expression de NEAT1 ;
un agent spécifique pour déterminer le niveau d'expression de CENPB ;
un agent spécifique pour déterminer le niveau d'expression de AA482221 ;
un agent spécifique pour déterminer le niveau d'expression de CPSF3 ; et
un agent spécifique pour déterminer le niveau d'expression d'un ou de plusieurs des gènes suivants :
tubuline, alpha1c
séryl-ARNt synthétase
stathmine-type 2
protéine du motif de fixation à l'ARN, liée à X
sous-unité 13 du complexe favorisant l'anaphase
réticulon 3
sous-unité du protéasome (prosome, macropaïne), type bêta, 3
LOC100509179 hypothétique
optineurine
protéine d'activation de tyrosine 3-monooxygénase/tryptophane 5-monooxygénase, polypeptide zêta
protéine 1 contenant le domaine OCIA
ATPase de transport de H+, sous-unité lysosomale E1 du V1 de 31 kDa
protéine de liaison intracellulaire (acide) du facteur de croissance des fibroblastes aftiphiline
protéine 2 de la lipodystrophie congénitale de Berardinelli-Seip (séipine)
transaminase glutamique oxalo-acétique 1, soluble (aspartate aminotransférase 1)
ATPase de transport de Ca++, muscle cardiaque, contraction lente de type 2
protéine 3 d'interaction de DAZ, en doigt de zinc
CAP, protéine 1 associée à l'adénylate cyclase
sous-complexe alpha de la NADH déhydrogénase (ubiquinone) 1, 7, 14,5 kDa
facteur 2B d'initiation de la traduction eucaryote, sous-unité gamma 3, 58 kDa
protéine ribosomique L15
tubuline, alpha 1b
sous-unité 26S du protéasome (prosome, macropaïne), non-ATPase, 1
sous-unité du protéasome (prosome, macropaïne), type bêta, 2
ATP synthase, de transport de H+, complexe F1 mitochondrial, polypeptide gamma 1 domaine 15 des répétitions tétratricopeptides
transformation du précurseur 4, sous-unité de la ribonucléase P/MRP (S. cerevisiae) rabaptine, RAB GTPase
protéine 1 effectrice de liaison ;
et
des instructions pour déterminer l'état de la maladie d'Alzheimer du sujet.

15. Utilisation d'un procédé dans lequel le niveau d'expression de TUBB, NEAT1, CENPB, AA482221 et CPSF3 est déterminé, lesdits niveaux d'expression étant utilisés comme moyen pour fournir une indication utile dans la distinction entre les sujets sains et les sujets atteints de la maladie d'Alzheimer.

16. Utilisation selon la revendication 15, dans laquelle le niveau d'expression d'un ou de plusieurs gène(s) énuméré(s) dans la revendication 2 est également déterminé pour fournir une indication utile dans la distinction entre les sujets sains et les sujets atteints de la maladie d'Alzheimer.

17. Procédé ou utilisation selon l'une quelconque des revendications précédentes, l'échantillon étant un échantillon de sang, de sérum, de liquide céphalorachidien, de salive, d'urine, de liquide tissulaire, de tissu, de tissu cérébral.

18. Procédé de sélection d'un sujet pour le traitement de la maladie d'Alzheimer ou pour l'étude approfondie de la maladie d'Alzheimer, comprenant les étapes consistant à :
i) déterminer le niveau d'expression de chacun de TUBB, NEAT1, CENPB, AA482221 et CPSF3, et facultativement d'un ou de plusieurs des biomarqueurs énumérés dans la revendication 2, dans un échantillon provenant du sujet ;
ii) déterminer si le niveau d'expression de chacun de TUBB, NEAT1, CENPB, AA482221 et CPSF3 est élevé ou réduit par rapport au niveau d'expression prévu de ce gène dans un échantillon comparable provenant d'un sujet sain,
le sujet étant sélectionné pour le traitement de la maladie d'Alzheimer ou pour l'étude approfondie de la maladie d'Alzheimer si le sujet est déterminé comme ayant des quantités élevées ou réduites de TUBB, NEAT1, CENPB, AA482221 et CPSF3.
